# EUROPEAN PATENT SPECIFICATION

(11) **EP 3 282 934 B1**
(45) Date of publication and mention of the grant of the patent: **09.09.2020**
(21) Application number: 16722342.9
(22) Date of filing: 15.04.2016
(51) Int. Cl.: A61B 5/022

(54) **AN IMPROVED BLOOD PRESSURE MEASUREMENT SYSTEM**
VERBESSERTES BLUTDRUCKMESSSYSTEM
SYSTÈME DE MESURE DE PRESSION SANGUINE AMÉLIORÉ

(30) Priority: 15.04.2015 GB 201506420
(43) Date of publication of application: 21.02.2018
(73) Proprietor: University of Newcastle Upon Tyne, Newcastle upon Tyne Tyne and Wear NE1 7RU (GB); A C Cossor & Son (Technology) Ltd, Harlow CM19 5QP (GB)
(72) Inventor: MURRAY, Alan, Newcastle upon Tyne Tyne and Wear NE2 3LA (GB); ZHENG, Dingchang, Newcastle upon Tyne Tyne and Wear NE7 7NN (GB); GRIFFITHS, Clive, Newcastle upon Tyne Tyne and Wear NE7 7RA (GB); LIU, Chengyu, Newcastle Upon Tyne Tyne and Wear NE1 7RU (GB)
(74) Representative: Secerna LLP
(86) International application number: PCT/GB2016/051065
(87) International publication number: WO 2016/166554

(56) References cited:
- EP-A1- 0 337 161
- US-A- 4 326 536
- US-A- 4 592 366
- US-A- 5 316 005
- US-A1- 2007 203 416

## Description

The present invention relates to an improved blood pressure measurement system which is able to measure actual blood pressures. More specifically the invention relates to a blood pressure monitor or sphygmomanometer which uses a new technique to detect high frequencies that exist only between systole and diastole, and thus identify micro-pulses associated with the opening and closing of an artery to achieve accurate blood pressure readings.

Examples of known blood pressure measurement systems are described in EP0337161 (Spacelabs Inc.), US2007/203416 (Lowe Andrew), and US5316005 (Tomita Mitsuei); all of these systems rely on the detection of Korotkoff sounds to enable the measurement of blood pressure.

High blood pressure (BP) is one of the leading cardiovascular risk factors for coronary artery disease, congestive heart failure, renal disease and stroke. Despite the importance of BP measurement and its very widespread use, it is generally accepted that it is one of the most poorly performed diagnostic measurements in clinical practice (from the American Heart Association (AHA), and British and European Hypertension Societies (BHS, ESH)) often due to either inadequate operator training or variability between devices.

Sphygmomanometers or blood pressure monitors are well known in the art. Typically they comprise an inflatable cuff, most commonly for positioning around a patient's upper arm at approximately heart level (although in some cases they can be positioned around a patient's wrist or finger), a pressure gauge or transducer for measuring cuff pressure and a mechanism for inflating the cuff to restrict blood flow. There is also a valve to allow controlled deflation of the cuff pressure. In some cases the inflatable cuff can take the form of a pressurisable chamber.

Traditional sphygmomanometers are manual and used by trained practitioners to measure and determine BP of a patient. These devices use a stethoscope for auscultation and require significant training. They must be used in quiet surroundings to allow the practitioner to hear the characteristic sounds, often referred to as Korotkoff sounds. Korotkoff sounds are the audible sounds heard with a stethoscope when it is placed over the brachial artery, distal to the sphygmomanometer's cuff. The sounds are typically heard when the cuff pressure is below systolic blood pressure (SBP) and above diastolic blood pressure (DBP), but suffer from the problem that these sounds can be heard outside this range, especially below DBP. They are associated with the starting and stopping of blood flow in the artery as the pulsatile blood pressure rises above and falls below the cuff pressure. As the cuff pressure is adjusted, over a range encompassing SBP and DBP, the appearance and disappearance of Korotkoff sounds allows SBP and DBP to be estimated. Manual sphygmomanometers of this type are considered to be the "gold standard" measurements providing the most accurate and reproducible readings.

More recently, automated electronic or digital sphygmomanometers have been developed and are commonly used both in doctors' surgeries, hospitals and at home by patients. Automated devices generally use electronic calculation of oscillometric measurements to determine BP rather than auscultation and as such can be used without significant training, unlike manual sphygmomanometers. They can also be used in a greater range of environments as it is not necessary for the environment to be quiet to obtain the reading. Some automated devices have attempted to use a microphone under the distal edge of the cuff to detect and analyse the Korotkoff sounds but accurate placement over the artery has been a problem. Attempts have been made to improve the efficiency of the automated detection method for Korotkoff sounds, but are complex and difficult to implement.

The oscillometric technique utilized by most automated devices is described briefly in this paragraph. The pulsatile pressure produced by the beating heart causes arteries to expand and contract. This effect is exaggerated when the artery is surrounded by a blood pressure cuff at a pressure between approximately SBP and DBP. The change in volume associated with this effect is transmitted through the soft, but incompressible, tissue of the arm to the inner wall of the cuff, making small changes to the volume of the enclosed gas in the bladder of the cuff. By Boyle's Law this superimposes low amplitude pulsatile changes onto the 'quasi-static' pressure in the cuff. For cuff pressure above SBP these pulsatile changes are relatively small, then between SBP and DBP rise steadily to a maximum (typically amplitude 1-2 mmHg) and then fall again to below DBP. The changes in amplitude are gradual and so accurate measurements of SBP and DBP are not possible. However arithmetic algorithms have been developed based on statistics which allow BPs to be estimated from the changing pattern of amplitudes. These techniques and the associated algorithms for determining BPs are well known in the art. In some cases the cuff pressures are increased in increments until the required BP readings are obtained, whilst in other cases the cuff is first taken to a high pressure then decreased in increments until the required readings are obtained. It is also possible to provide a smooth rather than incremental pressure reduction.

Although automated digital sphygmomanometers have significant ease of use benefits when compared to "gold standard" manual sphygmomanometers, it has been well documented that there are problems with the accuracy and reproducibility of their blood pressure readings. However, the high skills required to identify Korotkoff sounds using a manual sphygmomanometer has resulted in the popularity of the automated systems using the oscillometric technique, though sometimes an unreliable choice.

It is also known that it is often difficult to detect diastole correctly, even with the manual 'gold standard' sphygmomanometers. Traditionally, the systolic blood pressure is taken to be the pressure at which the first Korotkoff sound is first heard and the diastolic blood pressure is the pressure at which the Korotkoff sound is just barely audible; between systolic and diastolic blood pressure, the Korotkoff sounds peak and then begin to fade away. It is however very difficult, even for skilled practitioners, to determine when diastole occurs as the fading away of the Korotkoff sound can be very difficult to judge. In addition, if there is background noise this requires a skilled user with good hearing. The positioning of the stethoscope downstream of the cuff is also important and requires training for correct application. Attempts have been made to position the stethoscope under the cuff itself, at the downstream end of the cuff, in order to minimise movement of the stethoscope during use.

The following paragraphs describe the 'gold standard' auscultation method in further details. With the cuff of a sphygmomanometer placed around a patient's upper arm and inflated to a pressure above the patient's systolic blood pressure, and the stethoscope positioned downstream of the cuff over an artery, there is no audible Korotkoff sounds. This is because the pressure in the cuff is high enough such that it completely occludes the blood flow and the artery remains closed.

If the pressure is dropped to a level equal to that of the patient's systolic blood pressure, a first Korotkoff sound should be heard, although small changes in SBP may cause the sound to come and go on successive beats. Repetitive sounds for at least two consecutive beats allow cuff pressure to be considered as the systolic blood pressure. As the pressure in the cuff is allowed to fall further, thumping sounds or murmurs continue to be heard while the pressure in the cuff lies below the systolic and above the diastolic pressure.

Eventually, as the pressure in the cuff drops further, the sounds change in quality, then become muted, and finally disappear altogether. This occurs because, as the pressure in the cuff drops below the diastolic blood pressure, the cuff no longer provides restriction to blood flow allowing the blood flow to become smooth again and thus produce no further audible sound. The disappearance of sound is considered diastolic blood pressure. This "fading away" can be particularly difficult to recognise with it being very difficult to accurately determine when the sounds can no longer be heard.

At present, it is difficult to pinpoint when the actual opening and closing of the artery occurs using any of the current forms of sphygmomanometer.

It is an object of the present invention to obviate or mitigate one or more of the problems associated with both manual and automated sphygmomanometers.

According to a first aspect of the present invention there is a blood pressure measurement system comprising;
a cuff or a chamber which is attachable to apparatus selectively able to pressurise said cuff or chamber;
at least one pressure sensor in fluid communication with the cuff or chamber, said sensor able to sense cuff pressure and variances therein at least above 20Hz;
at least one pressure sensor in fluid communication with the cuff or chamber, said sensor able to sense cuff pressure and variances therein at least equal to or below 2 Hz; and
means to analyse the sensed pressure and variances, wherein said means;
   - analyses the pressure and variances therein at least above 20Hz to identify micro-pulses, wherein the micro-pulses comprise very low amplitude fast negative pressure deflections superimposed on a rising pressure component of an oscillometric waveform, and wherein the micro-pulses are associated with opening and closing of an artery and only exist between systolic blood pressure and diastolic blood pressure;
   - analyses the pressure and variances therein at least equal to or below 2Hz to determine a baseline cuff or chamber pressure; and
   - determines systolic blood pressure and diastolic blood pressure from the baseline cuff or chamber pressure in conjunction with the micro-pulses.

The new technique described here detects the actual opening and closing of the artery during pressure measurement, and so differs substantially from the two techniques described in the art. As it detects the fast high frequency snap arterial action, it may be termed the "arterial snap technique".

Said at least one sensor able to sense cuff pressure and variances therein of at least above 20Hz is capable of sensing the high frequency signals produced by the subtle pressure change associated with the artery opening and closing between systolic and diastolic blood pressure. The baseline cuff pressure is detected by said at least one sensor able to sense cuff pressure and variances therein at least below 2 Hz. This allows identification of SBP and DBP in conjunction with the high frequency micro-pulses detected by said at least one sensor.

It has been found that when cuff pressure was between SBP and DBP, superimposed on the rising pressure component of the oscillometric waveform a very low amplitude fast negative pressure deflection (amplitude of the order 0.05 mmHg) was observed. This pressure change immediately preceded the Korotkoff sound and was heard over the brachial artery near the elbow. This small micro-pulses disappeared when cuff pressure was above SBP and below DBP.

The origin for the observed micro-signal is believed to be as follows. When cuff pressure is between SBP and DBP, during each heart beat arterial pressure starts to rise. Initially the artery is occluded by the cuff pressure. When arterial pressure reaches cuff pressure, the 'dam bursts' or the 'flood gates open' and there is a sudden surge of blood through the previously occluded artery. This results in a net small, brief reduction in volume that is detected (by Boyle's law) as the very small amplitude, fast reduction in cuff pressure. The very small amplitude, fast reduction in pressure may in turn be detected by a very high sensitivity, high frequency response pressure sensor. This is immediately overtaken by continuation of the rising pressure of the oscillometric waveform as the previously occluded artery continues to fill.

This waveform may be further discriminated by appropriate signal processing including band pass filtering and because the main components of the signal are in the low audio bandwidth, a conventional sensitive microphone that can continue to function in the presence of high cuff pressure may provide a suitable detector. The waveform disappears above SBP (when the artery remains occluded) and below DBP (when the artery remains open) because there is no sudden surge and so it can provide a basis for accurate measurement of true blood pressures. Further, as the micro-signal can be detected by signal processing and computer algorithms via general principles known in the art, it provides the basis for an automated technique.

As such, the present invention does not rely on detection of Korotkoff sounds or estimates based on oscillometric techniques to determine BP and so obviates or at least mitigates the problems associated with manual and automated sphygmomanometers known in the art. Further, the present invention is non-invasive and relies on the detection of signals associated with the fluid in the cuff, which provides benefits over the auscultation methods described in the art.

Optionally there is a single pressure sensor that is able to sense cuff or chamber pressure and variances therein above 20Hz and sense cuff or chamber pressure and variances therein below 2Hz.

The low frequency pressure sensing allows measurement of the baseline cuff or chamber pressure giving a baseline pressure when pressurising/inflating or depressurising/deflating, whilst the higher frequency pressure sensing allows measurement of the snap micro-pulses identified by the inventors. As the system measures both high frequency variances in pressure and baseline cuff pressure it allows determination of micro pulses associated with systole and diastole.

Optionally said pressure sensor is able to sense both cuff or chamber pressure, and variances therein at least up to 100Hz.

Optionally said pressure sensor is able to sense both cuff or chamber pressure, and variances therein at least up to 300Hz.

It will be appreciated that in certain embodiments the sensors can read beyond the identified upper frequencies.

Optionally the reading from the pressure sensor is filtered electronically to remove components below 20 Hz or 30 Hz.

Preferably the pressure sensor is part of the cuff or chamber. Preferably the pressure sensor is integrated into a wall of the cuff or chamber.

Alternatively the pressure sensor is in close proximity to, or is proximal to, the cuff or chamber.

Alternatively, the system comprises a first high frequency pressure sensor and a second low frequency pressure sensor.

Alternatively the high frequency pressure sensor senses at least above 20Hz, or above, approximately 30Hz.

Alternatively the high frequency pressure sensor senses from approximately 20Hz, or approximately 30Hz, to approximately 300Hz.

The second low frequency pressure sensor may be able to sense cuff or chamber pressure and variances therein at least below 2Hz. Optionally the reading from the high frequency pressure sensor is filtered electronically to remove components below 20 Hz.

It will be appreciated that in certain embodiments the sensors can read beyond the identified upper and lower frequencies.

The blood pressure measurement system may comprise a classic band-pass analogue/digital filter between 20 and 300Hz, wherein the filtered signal is enhanced by the multiplication of a transfer function, reducing signals with low amplitude and enhancing the signals with large amplitude.

The reading from the high frequency pressure sensor may be filtered electronically to remove components below 30 Hz.

Preferably the low frequency pressure sensor and/or the high frequency pressure sensor is part of the cuff or chamber.

Alternatively the low frequency pressure senor and/or the high frequency pressure sensor is in close proximity to, or is proximal to, the cuff or chamber.

Optionally the system comprises a means for processing the information sensed by the pressure sensor.

The system may include means for storing the information from the pressure sensor.

Preferably the system comprises a display means for displaying information to a user.

The system may for example display real-time cuff pressure (as for a conventional device), stored 'micro-pulse' waveform, systolic blood pressure (SBP) and diastolic blood pressure (DBP), heart rate and/or heart rhythm.

Optionally a segment of high frequency cuff pressure change, which is defined from a fixed timing window referenced to its corresponding foot of the low frequency oscillometric pulse, is further processed using a low pass filter and the original segment is then replaced by the filtered segment for better BP determination.

Optionally, blood pressure readings can also be determined using the time difference information.

According to a second aspect of the present invention, there is provided a method for measuring blood pressure using a cuff or chamber, the method comprising:
- sensing high frequency signals of greater than at least 20Hz associated with a cuff or chamber pressure;
- sensing low frequency signals of equal to or less than at least 2 Hz associated with a baseline cuff or chamber pressure; and
- by means comprising a processor:
   - analysing the high frequency signals to identify micro-pulses, wherein the micro-pulses comprise very low amplitude fast negative pressure deflections superimposed on a rising pressure component of an oscillometric waveform, and wherein the micro-pulses are associated with opening and closing of an artery and only exist between systolic blood pressure and diastolic blood pressure;
   - analysing the low frequency signals to determine the baseline cuff or chamber pressure; and
   - determining systolic blood pressure and diastolic blood pressure from the baseline cuff or chamber pressure in conjunction with the micro-pulses.

The method may further comprise;
incrementally increasing a pressure of the cuff or chamber from below diastolic pressure; and wherein diastolic blood pressure is determined as the cuff or chamber pressure at which the high frequency micro-pulses are first detected, and systolic blood pressure is determined as being the cuff or chamber pressure at which the high frequency micro-pulses cease to be detected; or
incrementally or smoothly decreasing a pressure of a cuff or chamber from above systolic pressure; and wherein systolic blood pressure is determined as the cuff or chamber pressure at which the high frequency micro-pulses are first detected, and diastolic blood pressure is determined as being the cuff or chamber pressure at which the high frequency micro-pulses cease to be detected.

Preferably, the method further includes the step of sensing all high frequency signals of greater than at least 20 Hz between systolic and diastolic blood pressure. It is appreciated that between systolic and diastolic blood pressure when the artery is constricted, it opens and closes with every beat, creating high frequency signals, preferably sensed by cuff pressure and variances therein.

According to a third aspect of the present invention, there is provided use of the system of the first aspect of the present invention to measure blood pressure by sensing high frequency signals associated with systolic blood pressure and diastolic blood pressure.

The preceding discussion of the background to the invention is intended only to facilitate an understanding of the present invention. It should be appreciated that the material referred to was part of the common general knowledge as at the priority date of the application.

Throughout the description and claims of this specification, the words "comprise" and "contain" and variations of the words, for example "comprising" and "comprises", means "including but not limited to", and is not intended to (and does not) exclude other components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Throughout this document, reference to an automated sphygmomanometer relates to both fully automatic devices where a cuff or chamber is pressurised and depressurised e.g. by an electronically operated pump and valve, and to semiautomatic devices where the cuff or the chamber is inflated or pressurised by hand using a pumping bulb.

It should be noted that where reference is made to a cuff or an inflatable cuff a skilled person would understand that this could be a chamber or pressurisable chamber that itself may have no inner layer.

Features, integers or characteristics, and compounds described in conjunction with a particular aspect, embodiment or example of the invention are to be understood to be applicable to any other aspect, embodiment or example described herein unless incompatible therewith.

There now follows a description of a preferred embodiment(s) of the invention, by way of non-limiting example, with reference being made to the accompanying drawings, in which:
Figure 1 is a diagram showing a generic system layout for the system of the present invention;
Figure 2 (A and B): Examples of blood pressure measurement system set ups (left), and example of cuff pressure waveforms (top right of each window) and high frequency cuff pressure changes (right bottom of each window) recorded from the blood pressure cuff;
Figure 3: Three possible embodiments of high frequency (HF) pressure sensor location.
Figure 4: High frequency cuff pressure changes recorded with the sensor at two locations.
Figure 5: Processed high frequency cuff pressure changes. Top: Filtered high frequency cuff pressure changes using 30-300Hz band pass filter; Middle: with further processing of noise reduction (which may be required if noise is present; Bottom: with further process of enhancement.
   Note: This example clearly shows that more accurate BP determination could be achieved after further processing, from which the noises above SBP and below DBP are reduced.
   **Enhancement:** The filtered signal is processed by the multiplication of a transfer function. This process reduces the signals with low amplitude, and enhances the signals with relatively large amplitude.
   **Noise reduction:** At beat-by-beat level, one segment of high frequency cuff pressure change, defined from a fixed timing window referenced to its corresponding foot of the low frequency oscillometric pulse, is further processed for noise reduction using a low pass filter to better identify micro-pulses. The original segment is then replaced by the filtered segment.

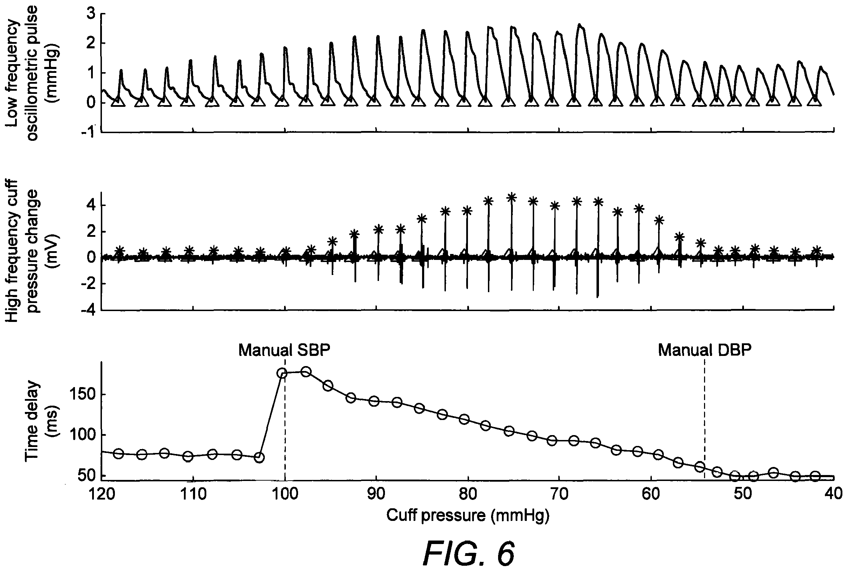

Figure 6: Low frequency oscillometic pulses (top), and cuff pressure changes (middle) that include components of both the high frequency micro-signal pulses and the oscillometric waveform, enabling the position of the arterial opening pulses relative to the oscillometric pulses to be more easily seen. On the bottom part of the figure, the time difference between the foot of the oscillometic pulses (marked with Δ) and the peaks of the cuff pressure changes signal (marked with *) are shown. Initially these times simply detect the leading edge of the oscillometric pulses, but as soon as SBP is reached the timing suddenly changes to the peak of the oscillometric pulse, and then continues to shorten until DBP is reached. Analysis of the high frequency snap micro-signal pulse relative to the oscillometric pulse enables a detection time window to be set, helping to exclude noise and making the detection of SBP and DBP more accurate in noisy conditions.

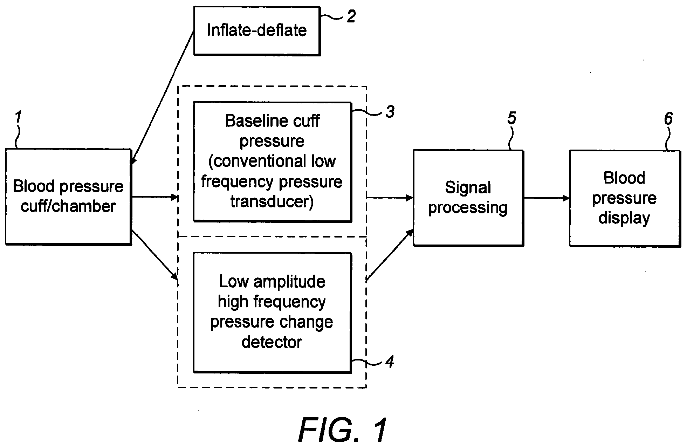

A general system according to the present invention is shown in Figure 1. The blood pressure cuff or chamber **1** may be a conventional cuff or may be of a custom design including that of a chamber without inner layer (the chamber typically having a lower internal volume c.f. a conventional cuff).

In a preferred embodiment the cuff or chamber incorporates a microphone, however this could instead be in close proximity to the cuff or chamber or in fact remote from the cuff or chamber, for example in a main device body which houses the processor. A low frequency pressure transducer is also present to read baseline cuff pressure. In certain embodiments a single pressure sensor or pressure transducer reads both low frequencies and high frequencies.

The system may include a means of inflating/deflating the cuff **2** or a means of pressurising/depressurising the chamber if that is being used. Cuff inflation/deflation of chamber pressurisation/depressurisation may be either manual or automated as is known from conventional sphygmomanometer technology. Alternatively such means **2** may not form part of the system but the system may be attachable to said means.

The system reads both baseline cuff pressure **3** and low amplitude high frequency pressure changes **4.**

The system may comprise additional signal processing **5** utilising hardware and/or software (which optionally may be included in the device body). This may include one or more of; a microphone amplifier, an analogue filter, a digital filter, digital discrimination, noise reduction means, micro pulse enhancement means, and pulse detection algorithms. Signal processing and enhancement can provide more accurate BP determination as "noises" above SBP and below DBP are reduced.

Finally, the recorded, and potentially further processed readings are displayed **6** to a user. Typically this will be via a visual display integrated with the processer, for example in a device body. The information could however be sent, using known data transfer technology, to a remote device such as a computer, laptop, mobile device for display or manipulation. As well as BP readings the following information could be obtained or determined and displayed; real-time cuff pressure (as for conventional device), stored 'micro pulse' waveform, SBP and DPB, heart rate and heart rhythm.

Determination of SBP and DBP can be carried out by;
manually positioning cursors on the displayed waveform trace of the micro pulse; automatic detection of SBP and DBP based on:
the appearance and disappearance of processed micro-pulses, or
time difference between initial hydraulic pulse (or oscillometric pulse foot) and micro- pulse
automatic detection with manual override.

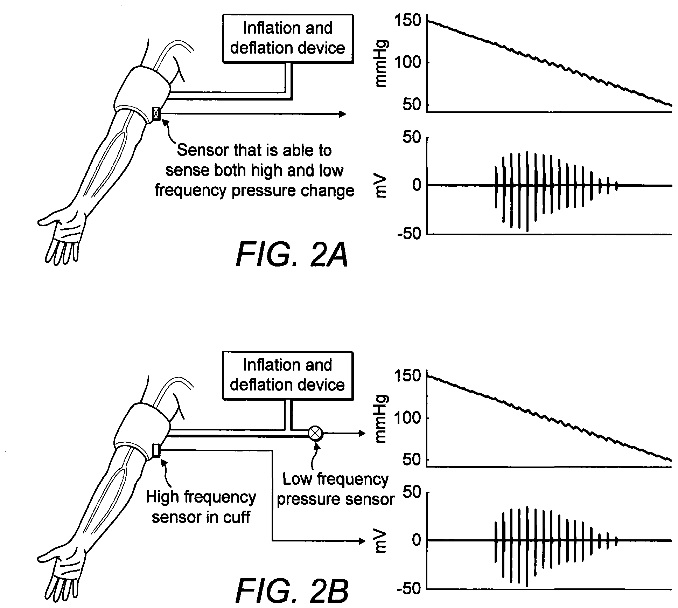

Using the system of the present invention, auscultation with a stethoscope is no longer required as accurate BP readings can be obtained based on the micro-pulses detected. Examples of the blood pressure measurement system set up alongside recorded waveforms are shown in Figure 2. Figure 2A shows a system where a cuff has a single pressure transducer or pressure sensor integrated into the wall of the cuff that is able to sense and record both low frequency (at least 0-2Hz) and high frequency (at least greater than >20Hz) pressure variances. Figure 2B shows a system where a cuff has a first high pressure transducer or pressure sensor integrated into the wall of the cuff that is able to sense and record high frequency (at least greater than >20Hz) pressure variances and a low frequency pressure transducer or pressure sensor that is able to sense and record low frequency (at least 0-2 Hz) remote from the cuff but in pneumatic communication therewith (for example in tubing associated with both the cuff and a means for inflating the same). In both 2A and 2B example cuff pressure waveforms are shown in the top right and example high frequency cuff pressure changes (micro-pulses) are shown in the bottom right.

The system directly senses the pressure variances in the air or fluid in the cuff or chamber (or "listens" to the sounds caused by changes in air or fluid pressure in the cuff). As the cuff now both acts as the site of restricted/released blood flow and the site of sensing pressure variances it is hypothesised that this allows the opening and closing of the artery to be more clearly sensed, in turn allowing systolic and diastolic blood pressure to be determined using the small micropulses that have been identified by the inventors using their direct readings. The system can therefore directly detect those pressure changes resulting from the artery opening and closing below the cuff, termed micropulses herein, and is not reliant on the arterial pulse producing an oscillometric pressure waveform (from which the blood pressures can only be estimated).

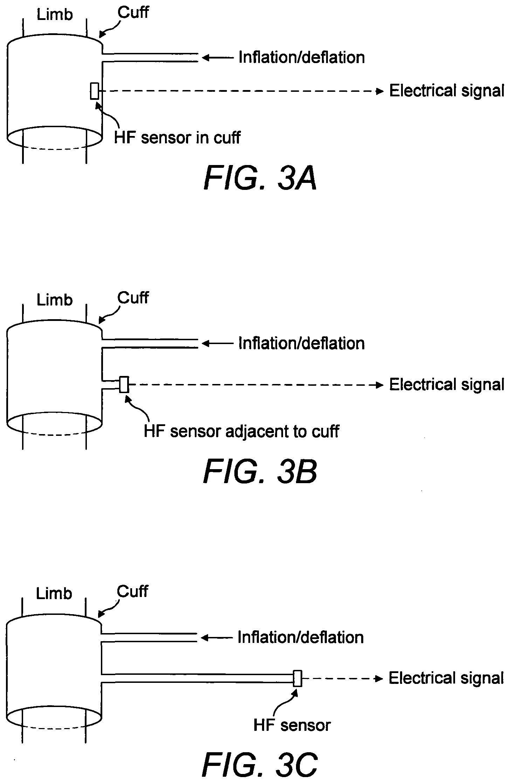

In use, a cuff (which could be replaced with an appropriate chamber) is placed around an individual's arm at approximately heart height. In one embodiment, the cuff comprises a pressure sensor in the form of a sensor located on the wall of the cuff. Figure 3 shows three further possible variants where a high frequency transducer or pressure sensor is located at different positions, however it would be understood by one skilled in the art that a single pressure sensor able to sense high and low frequencies could equally by placed in such positions.

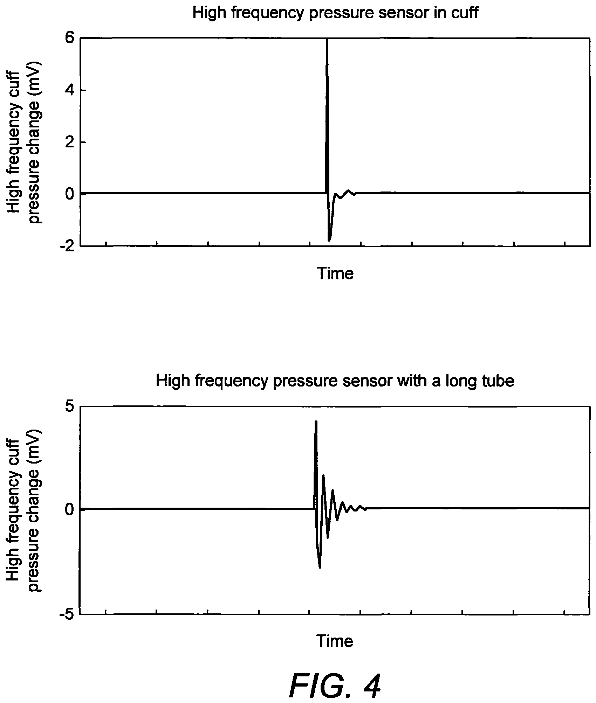

In a preferred embodiment the sensor is embedded into the wall of the cuff and heat sealed in place. The cuff is inflated to a pressure in excess of systolic pressure; this may be manually or under the control of a processor. Optionally, pulse wave amplitude value, heart rate, systolic blood pressure and various other parameters can be sensed and recorded during inflation of the cuff if so desired using standard methodologies such as the oscillometric method. It has been found that this particular embodiment where the high frequency pressure sensor (or combined high and low frequency pressure sensor) is integrated into the cuff (or chamber) provides a particularly clear signal. Figure 4 shows high frequency cuff pressure changes recorded with the sensor at two locations. When the sensor is remote from the cuff a resonant effect is observed whereas when the high frequency pressure sensor is in the cuff the resonant effect is not observed.

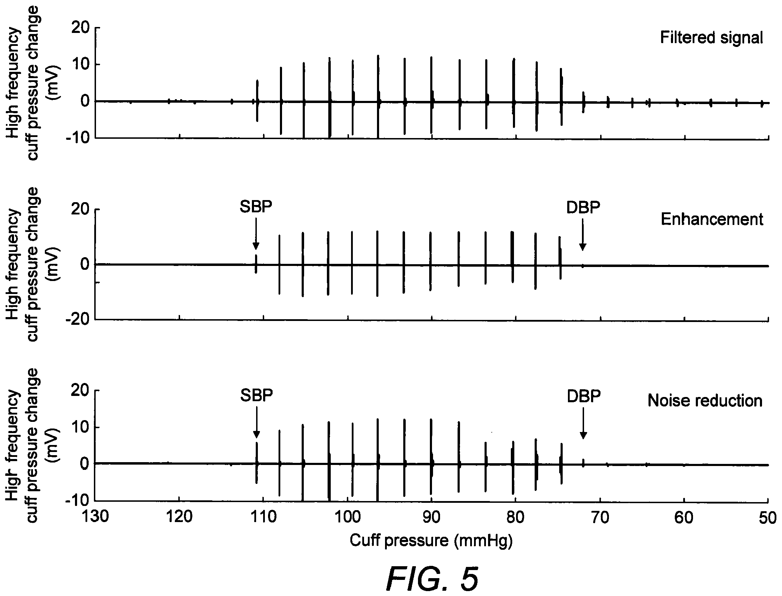

In certain embodiments, the reading(s) from the pressure sensor or sensors are further processed to give more accurate readings. Figure 5 shows how processing the variations in high frequency cuff pressure can provide very accurate BP determination. For example, the top graph shows filtered high frequency cuff pressure changes using 30-300Hz band pass filter. The middle graph shows the effect of further process of enhancement - The filtered signal is processed by the multiplication of a transfer function. This process reduces the signals with low amplitude, and enhances the signals with relatively large amplitude. The bottom graph show further processing with noise reduction - at beat-by-beat level, one segment of high frequency cuff pressure change, which is defined from a fixed timing window referenced to its corresponding foot of the low frequency oscillometric pulse, is further processed for noise reduction. The original segment is then replaced by the filtered segment. These graphs clearly show that more accurate BP determination can be achieved after further processing, from which the noises above SBP and below DBP are reduced.

Figure 6 shows how the signals can be used to determine BPs accurately using low frequency oscillometic pulses (top) and cuff pressure changes (middle) recorded from the cuff that include components of both the high frequency snap micro-signal pulses and the oscillometric waveform, enabling the position of the arterial opening pulses relative to the oscillometric pulses to be more easily seen.

In summary the present invention allows for accurate determination of true blood pressures. It will be understood that a preferred embodiment has at least the high frequency sensor, transducer incorporated into the wall of the cuff or chamber that is in contact with a person's arm in use - this provides particularly accurate readings. Further, the processing of the signal allows for very accurate results to be obtained with minimal requirements for user training.

## Claims

1. A blood pressure measurement system comprising;
a cuff or a chamber which is attachable to apparatus selectively able to pressurise said cuff or chamber;
at least one pressure sensor in fluid communication with the cuff or chamber, said sensor able to sense cuff or chamber pressure and variances therein at least above 20Hz;
at least one pressure sensor in fluid communication with the cuff or chamber, said sensor able to sense cuff or chamber pressure and variances therein at least equal to or below 2Hz; and
means to analyse the sensed pressure and variances, wherein said means is configured to:
- analyse the pressure and variances therein at least above 20Hz to identify micro-pulses, wherein the micro-pulses comprise very low amplitude fast negative pressure deflections superimposed on a rising pressure component of an oscillometric waveform, and wherein the micro-pulses are associated with opening and closing of an artery and only exist between systolic blood pressure and diastolic blood pressure;
- analyse the pressure and variances therein at least equal to or below 2Hz to determine a baseline cuff or chamber pressure; and
- determine systolic blood pressure and diastolic blood pressure from the baseline cuff or chamber pressure in conjunction with the micro-pulses.

2. A blood pressure measurement system as claimed in claim 1 wherein there is a single pressure sensor that is able to sense cuff or chamber pressure and variances therein above 20Hz and sense cuff or chamber pressure and variances therein below 2Hz; and/or wherein said pressure sensor is able to sense cuff or chamber pressure, and variances therein at least up to 100Hz.

3. A blood pressure measurement system as in claim 1 or claim 2 wherein said pressure sensor is able to sense cuff or chamber pressure, and variances therein at least up to 300Hz.

4. A blood pressure measurement system as in any of the previous claims wherein the reading from the pressure sensor is filtered electronically to remove components below 20 Hz or 30 Hz.

5. A blood pressure measurement system as in any of the previous claims wherein the pressure sensor is part of the cuff or chamber; and optionally wherein the pressure sensor is integrated into a wall of the cuff or chamber; or in close proximity to, or is proximal to, the cuff or chamber.

6. A blood pressure measurement system as in claim 1 wherein, the system comprises a first high frequency pressure sensor and a second low frequency pressure sensor.

7. A blood pressure measurement system as in claim 6 wherein the high frequency pressure sensor is configured to sense at least above 20 Hz, or above, approximately 30Hz.

8. A blood pressure measurement system as in claim 6 wherein the high frequency pressure sensor is configured to sense from approximately 20Hz or approximately 30Hz, to approximately 300Hz.

9. A blood pressure measurement system as in any of claims 6-8 wherein, the second low frequency pressure sensor is able to sense cuff or chamber pressure and variances therein at least below 2Hz; and optionally wherein the reading from the high frequency pressure sensor is filtered electronically to remove components below 20 Hz.

10. A blood pressure measurement system as in any one of the preceding claims comprising a classic band-pass analogue/digital filter between 20 and 300Hz, wherein the filtered signal is enhanced by the multiplication of a transfer function, reducing signals with low amplitude and enhancing the signals with large amplitude.

11. A blood pressure measurement system as in any of claims 6-10 wherein the reading from the high frequency pressure sensor is filtered electronically to remove components below 30 Hz.

12. A blood pressure measurement system as in any of claims 6 to 11 wherein the low frequency pressure sensor and/or the high pressure sensor is part of the cuff or chamber, or is close proximity to, or is proximal to, the cuff or chamber.

13. A blood pressure measurement system as in any of the previous claims wherein the system comprises a means for processing the information sensed by the at least one pressure sensor, and/or means for storing the information from the at least one pressure sensor, and/or means for displaying the information to a user.

14. A blood pressure measurement system as in any of the previous claims wherein a segment of high frequency cuff pressure change, which is defined from a fixed timing window referenced to its corresponding foot of a low frequency oscillometric pulse, is further processed using a low pass filter and the original segment is then replaced by a filtered segment.

15. A blood pressure measurement system as in any of claims 1 to 13 wherein blood pressure readings are determined using the time difference information.

16. A method for measuring blood pressure using a cuff or chamber, the method comprising:
- sensing high frequency signals of greater than at least 20Hz associated with a cuff or chamber pressure;
- sensing low frequency signals of equal to or less than at least 2 Hz associated with a baseline cuff or chamber pressure; and
- by means comprising a processor:
- analysing the high frequency signals to identify micro-pulses, wherein the micro-pulses comprise very low amplitude fast negative pressure deflections superimposed on a rising pressure component of an oscillometric waveform, and wherein the micro-pulses are associated with opening and closing of an artery and only exist between systolic blood pressure and diastolic blood pressure;
- analysing the low frequency signals to determine the baseline cuff or chamber pressure; and
- determining systolic blood pressure and diastolic blood pressure from the baseline cuff or chamber pressure in conjunction with the micro-pulses.

17. A method as claimed in claim 16 further comprising;
incrementally increasing a pressure of the cuff or chamber from below diastolic pressure; and wherein diastolic blood pressure is determined as the cuff or chamber pressure at which the high frequency micro-pulses are first detected, and systolic blood pressure is determined as being the cuff or chamber pressure at which the high frequency micro-pulses cease to be detected; or
incrementally or smoothly decreasing a pressure of a cuff or chamber from above systolic pressure; and wherein systolic blood pressure is determined as the cuff or chamber pressure at which the high frequency micro-pulses are first detected, and diastolic blood pressure is determined as being the cuff or chamber pressure at which the high frequency micro-pulses cease to be detected.

18. The method of any one of claims 16 to 17 further comprising the step of sensing all high frequency signals of greater than at least 20 Hz between systolic and diastolic blood pressure.

19. Use of the system of any of claims 1 to 15 to measure blood pressure by sensing high frequency signals associated with systolic blood pressure and diastolic blood pressure.

## Patentansprüche

1. Blutdruckmesssystem, umfassend:
eine Manschette oder eine Kammer, die an einer Vorrichtung angebracht werden kann, die selektiv die Manschette oder die Kammer mit Druck beaufschlagen kann;
mindestens einen Drucksensor in Fluidkommunikation mit der Manschette oder der Kammer, wobei der Sensor den Druck der Manschette oder der Kammer sowie von Veränderungen darin von mindestens über 20 Hz messen kann;
mindestens einen Drucksensor in Fluidkommunikation mit der Manschette oder der Kammer, wobei der Sensor den Druck der Manschette oder der Kammer sowie von Veränderungen darin von unterhalb oder gleich 2 Hz messen kann;
ein Mittel zum Analysieren des gemessenen Drucks und der Veränderungen, wobei das Mittel für folgende Zwecke gestaltet ist:
- Analysieren des Drucks und der Veränderungen darin von mindestens über 20 Hz, um Mikroimpulse zu identifizieren, wobei die Mikroimpulse schnelle Unterdruckausschläge mit sehr niedriger Amplitude umfassen, die eine ansteigende Druckkomponente einer oszillometrischen Kurvenform überlagern, und wobei die Mikroimpulse dem Öffnen und Schließen einer Arterie zugeordnet sind, und wobei sie nur zwischen systolischem Blutdruck und diastolischem Blutdruck existieren;
- Analysieren des Drucks und der Veränderungen darin von unterhalb oder gleich 2 Hz, um einen Baseline-Manschetten- oder Kammerdruck zu bestimmen; und
- Bestimmen des systolischen Blutdrucks und des diastolischen Blutdrucks anhand des Baseline-Manschetten- oder Kammerdrucks in Verbindung mit den Mikroimpulsen.

2. Blutdruckmesssystem nach Anspruch 1, wobei ein einzelner Drucksensor vorgesehen ist, der den Manschetten- oder Kammerdruck und Veränderungen darin oberhalb von 20 Hz messen kann, und der den Manschetten- oder Kammerdruck und Veränderungen darin unterhalb von 2 Hz messen kann; und/oder wobei der Drucksensor den Manschetten- oder Kammerdruck und Veränderungen darin von wenigstens bis zu 100 Hz messen kann.

3. Blutdruckmesssystem nach Anspruch 1 oder Anspruch 2, wobei der Drucksensor den Manschetten- oder Kammerdruck und Veränderungen darin von wenigstens bis zu 300 Hz messen kann.

4. Blutdruckmesssystem nach einem der vorstehenden Ansprüche, wobei der Messwert des Drucksensors elektronisch gefiltert wird, um Komponenten unterhalb von 20 Hz oder 30 Hz zu entfernen.

5. Blutdruckmesssystem nach einem der vorstehenden Ansprüche, wobei der Drucksensor eil der Manschette oder der Kammer ist; und wobei der Drucksensor optional in eine Wand der Manschette oder der Kammer integriert ist oder sich dicht an oder proximal zu der Manschette oder der Kammer befindet.

6. Blutdruckmesssystem nach Anspruch 1, wobei das System einen ersten Hochfrequenz-Drucksensor und einen zweiten Niederfrequenz-Drucksensor umfasst.

7. Blutdruckmesssystem nach Anspruch 6, wobei der Hochfrequenz-Drucksensor so gestaltet ist, dass er mindestens oberhalb von 20 Hz misst oder oberhalb von ungefähr 30 Hz.

8. Blutdruckmesssystem nach Anspruch 6, wobei der Hochfrequenz-Drucksensor so gestaltet ist, dass er von ungefähr 20 Hz oder von ungefähr 30 Hz bis ungefähr 300 Hz misst.

9. Blutdruckmesssystem nach einem der Ansprüche 6 bis 8, wobei der zweite Niederfrequenz-Drucksensor den Manschetten- oder Kammerdruck und Veränderungen darin wenigstens unterhalb von 2 Hz messen kann; und wobei der Messwert des Hochfrequenz-Drucksensors optional elektronisch gefiltert wird, um Komponenten unterhalb von 20 Hz zu entfernen.

10. Blutdruckmesssystem nach einem der vorstehenden Ansprüche, wobei dieses einen klassischen analogen/digitalen Bandpassfilter zwischen 20 und 300 Hz umfasst, wobei das gefilterte Signal durch die Multiplikation einer Übertragungsfunktion verstärkt wird, wobei Signale mit einer niedrigen Amplitude reduziert und Signale mit hoher Amplitude verstärkt werden.

11. Blutdruckmesssystem nach einem der Ansprüche 6 bis 10, wobei der Messwert des Hochfrequenz-Drucksensors elektronisch gefiltert wird, um Komponenten unterhalb von 30 Hz zu entfernen.

12. Blutdruckmesssystem nach einem der Ansprüche 6 bis 11, wobei der Niederfrequenz-Drucksensor und/oder der Hochdrucksensor Teil der Manschette oder der Kammer ist, oder dicht an oder proximal zu der Manschette oder der Kammer angeordnet ist.

13. Blutdruckmesssystem nach einem der vorstehenden Ansprüche, wobei das System ein Mittel zur Verarbeitung der durch den mindestens einen Drucksensor gemessenen Informationen umfasst und/oder ein Mittel zum Speichern der Informationen von dem mindestens einen Drucksensor und/oder ein Mittel zum Anzeigen der Informationen an einen Benutzer.

14. Blutdruckmesssystem nach einem der vorstehenden Ansprüche, wobei ein Teil der Hochfrequenz-Manschettendruckveränderung, der definiert ist ab einem festen Zeitgebungsfenster in Bezug auf dessen entsprechenden Boden eines oszillometrischen Niederfrequenzimpulses, unter Verwendung eines Tiefpassfilters weiterverarbeitet wird, und wobei der ursprüngliche Teil danach durch einen gefilterten Teil ersetzt wird.

15. Blutdruckmesssystem nach einem der Ansprüche 1 bis 13, wobei die Blutdruckmesswerte unter Verwendung von Zeitdifferenzinformationen bestimmt werden.

16. Verfahren zum Messen eines Blutdrucks unter Verwendung einer Manschette oder einer Kammer, wobei das Verfahren folgendes umfasst:
- Messen von Hochfrequenzsignalen von über mindestens 20 Hz, die einem Manschetten- oder Kammerdruck zugeordnet sind;
- Messen von Niederfrequenzsignalen von unterhalb oder gleich mindestens 2 Hz, die einem Baseline-Manschetten- oder Kammerdruck zugeordnet sind; und
- durch Mittel, die einen Prozessor umfassen:
- Analysieren der Hochfrequenzsignale, um Mikroimpulse zu identifizieren, wobei die Mikroimpulse schnelle Unterdruckausschläge mit sehr niedriger Amplitude umfassen, die eine ansteigende Druckkomponente einer oszillometrischen Kurvenform überlagern, und wobei die Mikroimpulse dem Öffnen und Schließen einer Arterie zugeordnet sind, und wobei sie nur zwischen systolischem Blutdruck und diastolischem Blutdruck existieren;
- Analysieren der Niederfrequenzsignale, um einen Baseline-Manschetten- oder Kammerdruck zu bestimmen; und
- Bestimmen des systolischen Blutdrucks und des diastolischen Blutdrucks anhand des Baseline-Manschetten- oder Kammerdrucks in Verbindung mit den Mikroimpulsen.

17. Verfahren nach Anspruch 16, ferner umfassend:
inkrementelles Erhöhen eines Drucks der Manschette oder der Kammer von einem Wert unterhalb des diastolischen Drucks; und wobei der diastolische Blutdruck bestimmt ist als der Manschetten- oder Kammerdruck, bei dem die Hochfrequenz-Mikroimpulse zuerst erkannt werden, und wobei der systolische Blutdruck bestimmt ist als der Manschetten- oder Kammerdruck, bei dem die Hochfrequenz-Mikroimpulse nicht mehr erkannt werden; oder
inkrementelles oder gleichmäßiges Verringern eines Drucks der Manschette oder der Kammer von einem Wert oberhalb des systolischen Drucks; und wobei der systolische Blutdruck bestimmt ist als der Manschetten- oder Kammerdruck, bei dem die Hochfrequenz-Mikroimpulse zuerst erkannt werden, und wobei der diastolische Blutdruck bestimmt ist als der Manschetten- oder Kammerdruck, bei dem die Hochfrequenz-Mikroimpulse nicht mehr erkannt werden.

18. Verfahren nach einem der Ansprüche 16 bis 17, das ferner den Schritt des Messens aller Hochfrequenzsignale von oberhalb mindestens 20 Hz zwischen dem systolischen und dem diastolischen Blutdruck umfasst.

19. Verwendung des Systems nach einem der Ansprüche 1 bis 15 zum Messen eines Blutdrucks durch Messen von Hochfrequenzsignalen, die dem systolischen Blutdruck und dem diastolischen Blutdruck zugeordnet sind.

## Revendications

1. Système de mesure de pression sanguine comprenant ;
un brassard ou une chambre qui peut être fixé à un appareil pouvant pressuriser sélectivement ledit brassard ou ladite chambre ;
au moins un capteur de pression en communication fluidique avec le brassard ou la chambre, ledit capteur pouvant détecter la pression de brassard ou de chambre et ses variations au moins supérieures à 20 Hz ;
au moins un capteur de pression en communication fluidique avec le brassard ou la chambre, ledit capteur pouvant détecter la pression de brassard ou de chambre et ses variations au moins égales ou inférieures à 2 Hz ; et
un moyen pour analyser la pression et les variations détectées, ledit moyen étant conçu pour :
- analyser la pression et ses variations au moins supérieures à 20 Hz pour identifier des micro-impulsions, les micro-impulsions comprenant des déviations rapides de pression négative de très faible amplitude superposées à une composante de pression croissante d'une forme d'onde oscillométrique, et les micro-impulsions étant associées à l'ouverture et à la fermeture d'une artère et n'existant qu'entre la pression sanguine systolique et la pression sanguine diastolique ;
- analyser la pression et ses variations au moins égales ou inférieures à 2 Hz pour déterminer une pression de base de brassard ou de chambre ; et
- déterminer la pression sanguine systolique et la pression sanguine diastolique à partir de la pression de base de brassard ou de chambre en conjonction avec les micro-impulsions.

2. Système de mesure de pression sanguine selon la revendication 1, un capteur de pression unique pouvant détecter la pression de brassard ou de chambre et ses variations supérieures à 20 Hz et détecter la pression de brassard ou de chambre et ses variations inférieures à 2 Hz ; et/ou ledit capteur de pression pouvant détecter la pression de brassard ou de chambre et ses variations au moins jusqu'à 100 Hz.

3. Système de mesure de pression sanguine selon la revendication 1 ou 2, ledit capteur de pression pouvant détecter la pression de brassard ou de chambre, et ses variations au moins jusqu'à 300 Hz.

4. Système de mesure de pression sanguine selon l'une quelconque des revendications précédentes, la lecture du capteur de pression étant filtrée électroniquement pour éliminer les composantes inférieures à 20 Hz ou 30 Hz.

5. Système de mesure de pression sanguine selon l'une quelconque des revendications précédentes, le capteur de pression faisant partie du brassard ou de la chambre ; et éventuellement le capteur de pression étant intégré dans une paroi du brassard ou de la chambre ; ou à proximité immédiate du brassard ou de la chambre, ou en étant proche.

6. Système de mesure de pression sanguine selon la revendication 1, le système comprenant un premier capteur de pression haute fréquence et un second capteur de pression basse fréquence.

7. Système de mesure de pression sanguine selon la revendication 6, le capteur de pression haute fréquence étant conçu pour détecter au moins plus de 20 Hz, ou plus, environ 30 Hz.

8. Système de mesure de pression sanguine selon la revendication 6, le capteur de pression haute fréquence étant conçu pour détecter d'environ 20 Hz ou d'environ 30 Hz à environ 300 Hz.

9. Système de mesure de pression sanguine selon l'une quelconque des revendications 6 à 8, le second capteur de pression basse fréquence pouvant détecter la pression de brassard ou de chambre et ses variations au moins inférieures à 2 Hz ; et éventuellement la lecture du capteur de pression haute fréquence étant filtrée électroniquement pour éliminer les composantes inférieures à 20 Hz.

10. Système de mesure de pression sanguine selon l'une quelconque des revendications précédentes, comprenant un filtre passe-bande analogique/numérique classique entre 20 et 300 Hz, le signal filtré étant amélioré par la multiplication d'une fonction de transfert, réduisant les signaux de faible amplitude et améliorant les signaux de grande amplitude.

11. Système de mesure de pression sanguine selon l'une quelconque des revendications 6 à 10, la lecture du capteur de pression haute fréquence étant filtrée électroniquement pour éliminer les composantes inférieures à 30 Hz.

12. Système de mesure de pression sanguine selon l'une quelconque des revendications 6 à 11, le capteur de pression basse fréquence et/ou le capteur de pression haute fréquence faisant partie du brassard ou de la chambre, ou étant à proximité immédiate du brassard ou de la chambre, ou en étant proche.

13. Système de mesure de pression sanguine selon l'une quelconque des revendications précédentes, le système comprenant un moyen pour traiter les informations détectées par l'au moins un capteur de pression, et/ou un moyen pour stocker les informations provenant de l'au moins un capteur de pression, et/ou un moyen pour afficher les informations à un utilisateur.

14. Système de mesure de pression sanguine selon l'une quelconque des revendications précédentes, un segment de changement de pression de brassard haute fréquence, qui est défini à partir d'une fenêtre de temps fixe référencée à son pied correspondant d'une impulsion oscillométrique basse fréquence, étant traité ultérieurement en utilisant un filtre passe-bas et le segment original étant ensuite remplacé par un segment filtré.

15. Système de mesure de pression sanguine selon l'une quelconque des revendications 1 à 13, les lectures de pression sanguine étant déterminées en utilisant les informations sur la différence de temps.

16. Procédé de mesure de pression sanguine à l'aide d'un brassard ou d'une chambre, le procédé comprenant les étapes consistant à :
- détecter des signaux haute fréquence supérieurs à au moins 20 Hz, associés à une pression de brassard ou de chambre ;
- détecter des signaux basse fréquence d'une fréquence égale ou inférieure à au moins 2 Hz, associés à une pression de base de brassard ou de chambre ; et
- par un moyen comprenant un processeur :
- analyser les signaux haute fréquence pour identifier les micro-impulsions, les micro-impulsions comprenant des déflexions rapides de pression négative de très faible amplitude superposées à une composante de pression croissante d'une forme d'onde oscillométrique, et les micro-impulsions étant associées à l'ouverture et à la fermeture d'une artère et n'existant qu'entre la pression sanguine systolique et la pression sanguine diastolique ;
- analyser les signaux basse fréquence pour déterminer la pression de base de brassard ou de chambre ; et
- déterminer la pression sanguine systolique et la pression sanguine diastolique à partir de la pression de base de brassard ou de chambre, en conjonction avec les micro-impulsions.

17. Procédé selon la revendication 16 comprenant en outre les étapes consistant à :
augmenter progressivement une pression du brassard ou de la chambre à partir d'une pression inférieure à la pression diastolique ; et la pression sanguine diastolique étant déterminée comme étant la pression de brassard ou de chambre à laquelle les micro-impulsions haute fréquence sont détectées pour la première fois, et la pression sanguine systolique étant déterminée comme étant la pression de brassard ou de chambre à laquelle les micro-impulsions haute fréquence cessent d'être détectées ; ou
diminuer progressivement ou régulièrement une pression d'un brassard ou d'une chambre à partir d'une pression systolique ; et la pression sanguine systolique étant déterminée comme étant la pression de brassard ou de chambre à laquelle les micro-impulsions haute fréquence sont détectées pour la première fois, et la pression sanguine diastolique étant déterminée comme étant la pression de brassard ou de chambre à laquelle les micro-impulsions haute fréquence cessent d'être détectées.

18. Procédé selon l'une quelconque des revendications 16 à 17 comprenant en outre l'étape consistant à détecter tous les signaux haute fréquence supérieurs à au moins 20 Hz entre la pression sanguine systolique et diastolique.

19. Utilisation du système selon l'une quelconque des revendications 1 à 15 pour mesurer la pression sanguine en détectant les signaux haute fréquence associés à la pression sanguine systolique et à la pression sanguine diastolique.
